(19) Europäisches Patentamt / European Patent Office / Office européen des brevets

(11) **EP 2 234 492 B1**

(12) **FASCICULE DE BREVET EUROPEEN**

(45) Date de publication et mention de la délivrance du brevet:
**15.06.2011 Bulletin 2011/24**

(21) Numéro de dépôt: **08872638.5**

(22) Date de dépôt: **19.12.2008**

(51) Int Cl.:
*A01N 47/02* *(2006.01)*    *A01N 25/02* *(2006.01)*
*A01P 7/04* *(2006.01)*    *A61K 31/415* *(2006.01)*
*A61K 47/34* *(2006.01)*    *A61P 33/14* *(2006.01)*
*A61K 9/00* *(2006.01)*    *A61K 47/22* *(2006.01)*

(86) Numéro de dépôt international:
**PCT/FR2008/001795**

(87) Numéro de publication internationale:
**WO 2009/103901 (27.08.2009 Gazette 2009/35)**

(54) **COMPOSITION PHARMACEUTIQUE CONTENANT UN DERIVE DE N-PHENYLPYRAZOLE ET DU GLYCOFUROL, UTILISATION POUR LA PREPARATION D'UN MEDICAMENT VETERINAIRE TOPIQUE POUR LUTTER CONTRE LES PUCES**

PHARMAZEUTISCHE ZUSAMMENSETZUNG MIT EINEM N-PHENYLPYRAZOLDERIVAT UND GLYCOFUROL, VERWENDUNG FÜR DIE HERSTELLUNG EINES TOPISCHEN TIERMEDIZINISCHEN MEDIKAMENTS GEGEN FÖHE

PHARMACEUTICAL COMPOSITION CONTAINING AN N-PHENYLPYRAZOLE DERIVATIVE AND GLYCOFUROL, USE FOR THE PREPARATION OF A TOPICAL VETERINARY MEDICAMENT FOR COMBATING FLEAS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.12.2007 FR 0709005**

(43) Date de publication de la demande:
**06.10.2010 Bulletin 2010/40**

(73) Titulaire: **VIRBAC**
**06516 Carros (FR)**

(72) Inventeur: **DERRIEU, Guy**
**F-06800 Cagnes Sur Mer (FR)**

(74) Mandataire: **Bernstein, Claire Jacqueline et al**
**Cabinet Orès**
**36, rue de Saint Pétersbourg**
**75008 Paris (FR)**

(56) Documents cités:
**WO-A-97/12521**

• **MERIAL ANIMAL HEALTH LIMITED: "Summary of product and characteristics. Frontline Spot On Cat" INTERNET ARTICLE, [Online] 2007, XP007905533 Extrait de l'Internet: URL: www.vmd.gov.uk/espcsite/Documents/1381 28.DOC> [extrait le 2008-08-26]**
• **LASHMAR U T ET AL: "TOPICAL APPLICATION OF PENETRATION ENHANCERS TO THE SKIN OF NUDE MICE: A HISTOPATHOLOGICAL STUDY" JOURNAL OF PHARMACY AND PHARMACOLOGY, PHARMACEUTICAL PRESS, vol. 41, 1 janvier 1989 (1989-01-01), pages 118-122, XP000945202 ISSN: 0022-3573**

**EP 2 234 492 B1**

**Description**

La présente Invention se rapporte à une composition pharmaceutique liquide renfermant, à titre de principe actif, un dérivé de N-phénylpyrazole et de l'alpha-(tetrahydrofuranyl)-omega-hydroxypoly(oxy-1,2-ethanediyl) à titre de solvant, ainsi que l'utilisation d'une telle composition pour la préparation d'un médicament vétérinaire antiparasitaire à application topique pour la prévention et/ou le traitement des infestations par les puces chez les animaux domestiques, en particulier chez les chiens et les chats.

Les animaux de compagnie sont souvent infestés par un ou plusieurs parasites se nourrissant de sang tels que les puces du chien ou du chat, les tiques, ou encore les gales.

Les puces sont des insectes dépourvus d'ailes, au corps comprimé latéralement et aux pattes très développées, adaptées au saut. Ce sont des ectoparasites, suceurs de sang de mammifères ou d'oiseaux. Les quelques 2000 espèces répertoriées appartiennent à l'ordre des siphonaptères. Deux espèces de puces sont communément rencontrées en Europe ; il s'agit de la puce du chat (*Ctenocephalides felis*) et de la puce du chien (*Ctenocephalides canis*) qui vivent dans la fourrure des animaux. La puce du chat, la plus fréquente, est capable de se reproduire à la fois sur le chat et le chien. Elle peut aussi s'attaquer à l'homme et aux autres animaux de compagnie, cependant le chat est le principal responsable d'infestation lorsque chats et chiens vivent dans le même environnement.

Les puces ont un cycle de vie complexe avec 4 stades distincts : oeuf, larve, nymphe et adulte. Elles s'accouplent dans les premières 8 à 48 heures suivant l'acquisition par l'hôte, après leur premier repas sanguin. Les femelles commencent ainsi à pondre 24 à 48 heures après ce premier repas sanguin. La puce adulte pond généralement sur l'animal. Les oeufs pondus sur l'animal n'y restent d'ailleurs pas et tombent sur le sol. En conditions optimales, la femelle peut pondre plus de 25 oeufs par jour. Elle en pondra plusieurs centaines durant toute sa vie. Après quelques jours, naît une larve vermiforme, blanche et poilue, d'environ 1,5 mm de long. La larve se nourrit de débris organiques, de dépouilles larvaires et du sang sec déféqué par les adultes. L'état larvaire dure de 1 à 3 semaines, si les conditions sont favorables (18° à 27°C et 70% d'humidité relative). La larve tisse ensuite un cocon et se nymphose. Normalement, la nymphe évolue en 1 à 2 semaines mais le passage à l'état adulte peut se prolonger jusqu'à 1 an, si les conditions sont défavorables. La puce adulte (petite et noire) émerge du cocon quand elle décèle vibrations, chaleur, concentration plus élevée en gaz carbonique, ce qui se produit lors du passage d'un chat, d'un chien... ou d'un homme ! Elle saute alors sur la victime, se nourrit aussitôt de sang et grossit rapidement en prenant une couleur plus claire d'un brun rougeâtre. La puce adulte vit de 6 à 12 mois. Sans nourriture, elle peut survivre jusqu'à 2 mois.

Les piqûres de puces provoquent des démangeaisons, chez l'animal comme chez l'homme. La salive de la puce (sécrétée à chaque piqûre) peut aussi, selon les individus, entraîner des réactions allergiques, immédiates ou retardées. Ces réactions se traduisent par diverses lésions cutanées et démangeaisons. On distingue deux types de dermatoses liées aux puces, à savoir la pulicose et la dermatite par allergie aux piqûres de puces. Si dans les deux cas la dermatose résulte d'une infestation plus ou moins importante par des puces, ce n'est que dans la seconde qu'un phénomène allergique est associé. La dermatite par allergie aux piqûres de puces (DAPP) est la cause la plus fréquente de prurit chez le chien. En France, chez le chien adulte, elle représente ainsi près de la moitié des dermatoses prurigineuses. Près de 80 % des chiens qui présentent une DAPP ont également une dermatite atopique. Réciproquement, deux chiens atopiques sur trois présentent une DAPP. Il est donc probable que les chiens atopiques soient prédisposés au développement d'une allergie aux piqûres de puces et que l'infestation par celles-ci soit un facteur déclenchant d'une dermatite atopique. Cela justifie de la nécessité d'un contrôle antipuce très poussé chez les chiens atopiques ou appartenant à des races à risque. De plus, la DAPP est probablement la principale cause de réapparition du prurit chez les chiens atopiques désensibilisés.

Les puces du genre *Ctenocephalides* sont par ailleurs des hôtes intermédiaires de *Dipylidium caninum,* qui est un vers parasite de l'intestin grêle du chien et du chat. Le carnivore s'infeste en avalant les puces parasitées. Cette infestation peut provoquer un prurit anal, un engorgement des sacs anaux, ainsi qu'une dermatite de la région périnéale. C'est pourquoi, il est parfois conseillé de vermifuger régulièrement les animaux en complément de la lutte contre les puces.

De la même manière, les tiques (*Rhipicephalus sp., Ixodes sp., Dermacentor sp., Amblyomma sp.,* ...) peuvent aussi causer un stress à l'animal et nuire à sa santé. Elles peuvent aussi nuire à l'homme. Mais le plus grave problème des tiques est qu'elles sont un vecteur d'agents pathogènes pouvant concerner l'animal autant que l'homme. Parmi les maladies majeures devant être évitées, on peut citer les Borellioses (maladie de Lyme à *Borellia burgdorferi*), les Babésioses (piroplasmoses *à Babesia sp.*), et les Rickettsioses. Les tiques peuvent aussi libérer des toxines aux propriétés paralysantes et inflammatoires, et parfois mortelles.

La galle (*Demodex sp., Sarcoptes sp., Otodectes sp.,* ...) est particulièrement difficile à combattre car il existe très peu de matières actives efficaces. Elle requiert des traitements fréquents.

Les infestations par ces différents parasites, et tout particulièrement par les puces, représentent donc un important problème sanitaire pour les animaux qui sont infestés et imposent de pouvoir disposer de traitements adaptés. Il convient en particulier que le traitement ait non seulement une efficacité immédiate (rapidité d'action) mais également une efficacité prolongée dans le temps (rémanence) afin d'éviter, d'une part, les traitements répétés et, d'autre part, tout risque d'infestation et/ou de réinfestation pendant une période prolongée. La puce, en particulier, doit être éliminée avant qu'elle ne se reproduise et commence à pondre.

Il existe beaucoup substances insecticides plus ou moins actives et plus ou moins coûteuses. Des phénomènes de résistance apparaissent liés à leur utilisation, c'est notamment le cas lors de l'utilisation des carbamates, des composés organophosphorés et des pyréthroïdes.

Par ailleurs, les demandes de brevets EP 0 295 117 et EP 0 352 944 décrivent une grande famille de N-phénylpyrazoles ayant un très large spectre d'activité, y compris des activités antiparasitaires.

Bien qu'efficaces, les dérivés de N-phénylpyrazoles, et en particulier le 5-amino-1-[2,6-dichloro-4-(trifluorométhyl)phényl]-4-(trifluorométhylsulfinyl)-1H-pyrazole-3-carbonitrile (fipronil), sont parfois difficiles à formuler car ils ne présentent pas toujours une solubilité suffisante dans les excipients classiquement utilisés pour la préparation de compositions antiparasitaires liquides prêtes à l'emploi.

En effet, les produits actifs contre les parasites suceurs de sang, et en particulier contre les puces, peuvent se présenter notamment sous la forme de compositions liquides (pipettes) ou solutions cutanées pour dépôt ou encore "Spot-On", à appliquer très facilement, en une seule fois, de façon topique directement sur la peau de l'animal, généralement entre les omoplates.

Cependant, dans ce type de composition, le fipronil est souvent difficile à formuler et peut conduire à des phénomènes de cristallisation. Afin de surmonter ce problème, il a déjà été proposé, notamment dans la demande de brevet EP 0 881 881, de formuler les dérivés de N-phénylpyrazoles en milieu solvant en présence d'un inhibiteur de cristallisation. Le produit Frontline® Spot-On Chat et Chien, vendu en France par la société Merial SAS s'inscrit dans cette technologie.

De telles compositions, si elles sont bien adaptées pour éviter les problèmes de cristallisation de ces principes actifs particuliers, ne donnent cependant pas toujours entière satisfaction d'un point de vue de la durée de protection qu'elles confèrent à l'animal. Dans le cas du produit Frontline® Spot-On Chat et Chien notamment, la durée de protection contre les nouvelles infestations par les puces annoncée par le fabricant est limitée à 4 semaines chez le chat et à 2 mois chez le chien. Cependant, des essais d'efficacité anti-parasitaires conduits selon les standards actuels ne permettent pas de reproduire ces résultats d'efficacité prolongée et le produit ne dispose donc pas toujours d'une rémanence totalement satisfaisante.

C'est donc afin de remédier à l'ensemble des problèmes rencontrés avec les produits antiparasitaires actuellement disponibles sur le marché et de pourvoir à un produit permettant de prévenir et de traiter efficacement les infestations par les puces chez les animaux domestiques, aussi bien chez le chat que chez le chien, que les Inventeurs ont mis au point ce qui fait l'objet de l'Invention. Ils se sont en particulier donnés pour but de pourvoir à un produit pour la prévention et le traitement des infestations par les puces chez les animaux domestiques qui soit facile à formuler, facile à administrer, tout en ayant une action rapide et plus rémanente que les produits actuellement disponibles sur le marché.

Un autre objectif de l'invention est de fournir de telles compositions facilement utilisables quelles que soient l'espèce animale, la taille de l'animal ou la nature de son pelage.

Un autre objectif de l'invention est de disposer de compositions efficaces ne nécessitant pas de mouiller tout l'animal.

Ces objectifs sont atteints par la composition pharmaceutique antiparasitaire qui fait l'objet de la présente invention.

De manière inattendue, la composition pharmaceutique antiparasitaire de l'invention fournit une activité efficace et prolongée dans le traitement et la protection des animaux domestiques sous forme de solution prête à l'emploi d'utilisation aisée. Elle permet également de formuler le fipronil sans observer de phénomène de cristallisation.

Ainsi, la présente Invention a pour objet une composition pharmaceutique liquide, caractérisée par le fait qu'elle renferme :

- à titre de principe actif, le 5-amino-1-[2,6-dichloro-4-(trifluorométhyl)phényl]-4-(trifluorométhylsulfinyl)-1H-pyrazole-3-carbonitrile (fipronil), et
- à titre de solvant, de l'alpha-(tetrahydrofuranyl)-omega-hydroxypoly(oxy-1,2-ethanediyl).

L'alpha-(tetrahydrofuranyl)-omega-hydroxypoly(oxy-1,2-ethanediyl), également dénommé glycofurol, est un solvant bien connu dans l'industrie pharmaceutique ; il est cependant habituellement utilisé comme solvant dans des compositions pharmaceutiques à administrer par voie injectable.

Selon l'Invention, ladite composition pharmaceutique est en particulier destinée à être administrée chez le chat ou chez le chien.

Au sein de la composition pharmaceutique utilisée conformément à l'Invention, le fipronil représente de préférence de 1 à 20 g environ pour 100 ml de composition, et encore plus préférentiellement de 5 à 15 g environ pour 100 ml de composition. Il doit être bien entendu, toutefois, que ces quantités sont données à titre indicatif et qu'elles peuvent être modulées selon les besoins de la formulation notamment eu égard aux doses efficaces en fonction de l'animal à traiter et de son poids.

La composition pharmaceutique utilisée conformément à l'Invention peut en outre renfermer un ou plusieurs excipients pouvant par exemple être choisis parmi les agents tensioactifs, les agents épaississants, les colorants, les parfums, les antioxydants parmi lesquels on peut citer à titre d'exemple non limitatif le butylhydroxyanisole, le butylhydroxytoluène, le gallate de propyle, le palmitate d'ascorbyle, les extraits de romarin et leurs mélanges.

Lorsqu'il(s) est (sont) présent(s), le ou les agents antioxydants représentent de préférence de 0,005 à 2 % en poids environ, et encore plus préférentiellement de 0,01 à 0,1 % en poids environ par rapport au volume total de la composition.

Outre le fipronil, la composition pharmaceutique peut comprendre également un ou plusieurs principes actifs antiparasitaires additionnels. A titre de principe actif antiparasitaire additionnel, on peut notamment mentionner les acaricides tels que l'amitraz ou le cymiazole, les inhibiteurs du développement des puces et des tiques, souvent appelés "IGR" pour "Insect Growth Regulators" en anglais, tels que le pyriproxyfène et l'éthoxazole, les endoparasiticides tels que les avermectines et leurs dérivés comme par exemple l'ivermectine, l'abamectine, la doramectine et la moxydectine, les milbémycines, ainsi que les composés actifs contre les phlébotomes et les ectoparasites des animaux domestiques.

De telles combinaisons d'actifs peuvent être utiles en vue d'élargir le spectre d'action de la composition conforme à la présente invention.

La composition pharmaceutique conforme à l'Invention peut facilement être préparée par simple dilution du fipronil et éventuellement du ou des principes actifs antiparasitaires additionnels dans le glycofurol.

Après sa préparation, la composition pharmaceutique est de préférence conditionnée en pipettes monodose.

Un autre objet de la présente demande est l'utilisation d'une composition pharmaceutique liquide telle que décrite précédemment pour la préparation d'un médicament vétérinaire antiparasitaire à application topique pour la prévention (protection) et/ou le traitement des infestations par les puces chez les animaux domestiques, en particulier chez les chiens ou les chats.

Selon cette utilisation, ledit médicament est destiné à être appliqué par application directe sur la peau de l'animal, au niveau des omoplates ou sur une ligne dorsale partant de la base de la queue et remontant jusqu'au cou.

La quantité de médicament à administrer peut varier de 0,3 à 1,5 ml environ, de préférence de 0,5 ml environ, chez le chat et de 0,3 à 6,0 ml environ chez le chien, en fonction du poids de l'animal considéré et de la posologie.

Le volume à appliquer selon l'invention doit correspondre de préférence à une dose unitaire de fipronil allant de 0,3 à 60 mg par kg de poids corporel, et encore plus préférentiellement de 5 à 15 mg par kg de poids corporel.

Ainsi selon une forme de réalisation préférée de l'invention, ledit médicament est destiné à administrer une dose unitaire de fipronil allant de 0,3 à 60 mg par kg de poids corporel, et encore plus préférentiellement de 5 à 15 mg par kg de poids corporel.

Outre les dispositions qui précèdent, l'Invention comprend encore d'autres dispositions qui ressortiront de la description qui va suivre, qui se réfère à un exemple de préparation d'une composition pharmaceutique conforme à l'invention, ainsi qu'à un exemple mettant en évidence l'efficacité de ladite composition vis-à-vis du traitement des puces chez le chien.

Il doit être bien entendu toutefois que ces exemples sont donnés uniquement à titre d'illustration de l'objet de l'Invention, dont ils ne constituent en aucune manière une limitation.

**EXEMPLE 1 : ÉTUDE DE L'EFFICACITÉ D'UNE COMPOSITION CONFORME A L'INVENTION CONTRE LES PUCES CHEZ LE CHIEN**

Dans cet exemple, on a réalisé une étude destinée à déterminer et à comparer l'efficacité de deux compositions topiques à base de fipronil :

- une composition A conforme à l'invention constituée de fipronil et de glycofurol ;
- le produit commercialisé sous la dénomination Frontline® Top Spot par la société Mérial.

**1) Matériel et Méthodes**

a) Type d'étude

Il s'agit d'une étude d'efficacité contrôlée en aveugle, randomisée, réalisée en parallèle sur 3 groupes de six chiens.

b) Animaux utilisés et conditions de maintenance

Les chiens utilisés dans cette étude étaient des chiens bâtards appartenant à l'espèce *Canis familiaris,* males ou femelles, âgés de plus de 6 mois, pesant entre 6 kg et 25 kg. Avant le début de l'étude, il a été vérifié que tous les chiens étaient en bonne santé et qu'ils n'étaient pas infestés de puces. Tous les chiens ont été vermifugés et acclimatés aux conditions de vie pendant au moins 7 jours avant le démarrage de l'étude.

Il a également été vérifié que les chiens n'avaient reçu aucun traitement topique contre les puces au cours de 12 semaines précédent le début de l'étude.

Pendant la période d'acclimatation et toute la durée de l'étude, les chiens ont été gardés à l'intérieur dans une pièce climatisée, chaque chien étant confiné dans un enclos individuel de dimensions 1,9 m x 2,97 m sans litière et sans contact possible entre les différents chiens engagés dans l'étude. Le numéro d'identification, le numéro de groupe et le type de composition administrée a été noté à l'extérieur de chaque enclos. La température de la pièce a été maintenue à environ 20°C $\pm$ 4 °C. Les chiens ont été soumis à une alternance de 12 heures de lumière et de 12 heures d'obscurité.

Les animaux ont été nourris une fois par jour avec des croquettes pour chien du commerce vendues sous la dénomination commerciale Ultradog Superwoof par la société Nola, une division de Foodcorp., selon les recommandations du fabricant, et ils ont eu de l'eau potable fraîche à volonté.

c) Compositions testées

On a préparé la composition A suivante conforme à l'invention :

| | |
|---|---|
| - Fipronil | 10 g |
| - Butylhydroxyanisole | 0,02 g |
| - Butylhydroxytoluène | 0,01 g |
| - Glycofurol q.s. | 100 ml |

Cette composition a été comparée au produit Frontline® Top Spot vendu par la société Merial, contenant 10 % (g/100 ml) de fipronil et un mélange d'excipients. Il a été utilisé tel que fourni par le fabriquant.

**d) Traitements**

Groupe 1 : Traitement avec la composition A à raison de 0,067 ml par kg de poids corporel,
Groupe 2 : Traitement avec le produit Frontline® Top Spot à raison de 0,067 ml par kg de poids corporel,
Groupe 3 : Contrôle négatif : pas de traitement,

Le traitement a été appliqué de façon topique, entre les omoplates des chiens, en une seule fois au commencement de l'étude (J = 0).

e) Infestations par les puces / Mesure de l'efficacité des traitements

6 jours avant le commencement de l'étude (J = -6), tous les chiens ont été infestés par environ 100 puces de laboratoire, souche *Ctenocephalis felis,* de sexe male ou femelle. Les puces ont ensuite été comptées 5 jours avant le début du traitement (J = -5). Pour ce faire, toutes les puces présentes sur un animal sont récoltées par peignage du chien puis comptées après peignage. Le nombre de puces est ainsi déterminé. Après comptage, et avant administration du traitement les puces sont remises sur l'animal.

Il a ensuite été procédé au comptage du nombre de puces encore vivantes 1 jour après l'administration de la composition (J = 1).

Les chiens ont à nouveau été infestés par une quantité connue de puces (environ 100) 7 jours (J = 7), 14 jours (J = 14), 21 jours (J = 21), 35 jours (J = 35), 42 jours (J = 42), 49 jours (J = 49) et 56 jours (J = 56) après l'administration du traitement.

Un comptage des puces encore vivantes a alors été effectué 24 heures après chacune de ces nouvelles infestations (J = 8 ; J = 15; J = 22 ; J = 36 ; J = 43 ; J = 50 et J = 57).

A chaque comptage, l'efficacité du traitement a été calculée selon l'équation suivante :

$$\text{\% d'efficacité} = 100 \text{ x } (NP_vC - NP_vT) / NP_vC$$

dans laquelle :

- $NP_vC$ est la moyenne géométrique du nombre de puces vivantes comptées sur les chiens du groupe 3 (contrôle) ;
- $NP_vT$ est la moyenne géométrique du nombre de puces vivantes comptées sur les chiens d'un groupe ayant reçu un traitement (Groupe 1 ou 2).

Un traitement est dit efficace si le pourcentage d'efficacité est supérieur ou égal à 95%.

**2) Résultats**

Les résultats moyens obtenus sont reportés dans le tableau I ci après :

**TABLEAU I**

| Jours | GROUPE 1 (Composition A) | GROUPE 2 (Frontline ® Top Spot) |
|---|---|---|
| J = 1 | 93.6 | 84.4 |
| J = 8 | 99.1 | 99.8 |
| J = 15 | 99.8 | 99.8 |
| J = 22 | 99.8 | 100.0 |
| J = 29 | 99.5 | 98.7 |
| J = 36 | 99.4 | 96.9 |
| J = 43 | 98.8 | 96.8 |
| J = 50 | **97.0** | **93.7** |
| J = 57 | **92.9** | **74.8** |

Ces résultats montrent que :

- la composition A agit plus rapidement que le produit Frontline® Top Spot (comparaison des % d'efficacité à J = 1) ;
- la composition A conforme à la présente invention reste efficace pendant 7 semaines (J = 50) contre les infestations par les puces chez le chien ;
- le produit Frontline ® Top Spot reste efficace pendant 6 semaines (J = 43) contre les infestations par les puces chez le chien.

La rapidité d'action et la meilleure rémanence de la composition A conforme à la présente Invention sont ainsi clairement démontrées.

**Revendications**

1. Composition pharmaceutique liquide, **caractérisée par le fait qu'**elle renferme :

   - à titre de principe actif, le 5-amino-1-[2,6-dichloro-4-(trifluorométhyl)phényl]-4-(trifluorométhylsulfinyl)-1H-pyrazole-3-carbonitrile représentant de 1 à 20 g pour 100 ml de composition, et
   - à titre de solvant, de l'alpha-(tetrahydrofuranyl)-omega-hydroxypoly(oxy-1,2-ethanediyl).

2. Composition selon la revendication 1, **caractérisée par le fait que** le 5-amino-1-[2,6-dichloro-4-(trifluorométhyl)phényl]-4-(trifluorométhylsulfinyl)-1H-pyrazole-3-carbonitrile représente de 5 à 15 g pour 100 ml de composition.

3. Composition selon l'une quelconque des revendications précédentes, **caractérisée par le fait qu'**elle renferme en outre un ou plusieurs excipients choisis parmi les agents tensioactifs, les agents épaississants, les colorants, les parfums et les antioxydants.

4. Composition selon la revendication 3, **caractérisée par le fait que** les antioxydants sont choisis parmi le butylhydroxyanisole, le butylhydroxytoluène, le gallate de propyle, le palmitate d'ascorbyle, les extraits de romarin et leurs mélanges.

5. Composition selon la revendication 3 ou 4, **caractérisée par le fait que** lorsqu'ils sont présents, le ou les antioxydants représente de 0,005 à 2 % en poids par rapport au volume total de la composition.

6. Composition selon l'une quelconque des revendications précédentes, **caractérisée par le fait qu'**elle comprend un ou plusieurs principes actifs antiparasitaires additionnels.

7. Composition selon la revendication 6, **caractérisée par le fait que** les principes actifs antiparasitaires additionnels sont choisis parmi les acaricides, les inhibiteurs du développement des puces et des tiques, les endoparasiticides et les principes actifs contre les phlébotomes et les ectoparasites des animaux domestiques.

8. Utilisation d'une composition pharmaceutique liquide telle que décrite à l'une quelconque des revendications précédentes, pour la préparation d'un médicament vétérinaire antiparasitaire à application topique pour la prévention et/ou le traitement des infestations par les puces chez les animaux domestiques.

9. Utilisation selon la revendication 8, **caractérisée par le fait que** ledit médicament est destiné aux chiens ou aux chats.

10. Utilisation selon la revendication 8 ou 9, **caractérisée par le fait que** ledit médicament est destiné à être administré en une quantité variant de 0,3 à 1,5 ml chez le chat.

11. Utilisation selon la revendication 8 ou 9, **caractérisée par le fait que** ledit médicament est destiné à être administré en une quantité variant de 0,3 à 6,0 ml chez le chien.

12. Utilisation selon l'une quelconque des revendications 8 à 11, **caractérisée par le fait que** ledit médicament est destiné à administrer une dose de unitaire de fipronil allant de 5 à 15 mg par kg de poids corporel.

## Claims

1. A liquid pharmaceutical composition, **characterized in that** it contains:

   - 5-amino-1-[2,6-dichloro-4-(trifluoromethyl)-phenyl]-4-(trifluoromethylsulfinyl)-1H-pyrazole-3-carbonitrile as active ingredient represents an amount ranging from 1 to 20 g per 100 ml of composition, and
   - alpha-(tetrahydrofuranyl)-omega-hydroxypoly(oxy-1,2-ethanediyl) as solvent.

2. The composition as claimed in claim 1, **characterized in that** the 5-amino-1-[2,6-dichloro-4-(trifluoromethyl)phenyl]-4-(trifluoromethylsulfinyl)-1H-pyrazole-3-carbonitrile represents from 5 to 15 g per 100 ml of composition.

3. The composition as claimed in any one of the preceding claims, **characterized in that** it also contains one or more excipients chosen from surfactants, thickeners, dyes, fragrances and antioxidants.

4. The composition as claimed in claim 3, **characterized in that** the antioxidants are chosen from butylhydroxyanisole, butylhydroxytoluene, propyl gallate, ascorbyl palmitate and extracts of rosemary, and mixtures thereof.

5. The composition as claimed in claim 3 or 4, **characterized in that**, when it (they) is (are) present, the antioxidant (s) represent(s) from 0.005% to 2% by weight, relative to the total volume of the composition.

6. The composition as claimed in any one of the preceding claims, **characterized in that** it comprises one or more additional antiparasitic active ingredients.

7. The composition as claimed in claim 6, **characterized in that** the additional antiparasitic active ingredients are chosen from acaricides, insect growth regulators for fleas and ticks, endoparasiticides and active ingredients that are active against sandflies and ectoparasites of domestic animals.

8. The use of a liquid pharmaceutical composition as described in any one of the preceding claims, for the preparation of an antiparasitic veterinary medicament for topical application, for the prevention and/or treatment of infestations with fleas in domestic animals.

9. The use as claimed in claim 8, **characterized in that** said medicament is intended for dogs or cats.

10. The use as claimed in claim 8 or 9, **characterized in that** said medicament is intended to be administered in an amount ranging from 0.3 to 1.5 ml in cats.

11. The use as claimed in claim 8 or 9, **characterized in that** said medicament is intended to be administered in an amount ranging from 0.3 to 6.0 ml in dogs.

12. The use as claimed in any one of claims 8 to 11, **characterized in that** said medicament is intended to be administered at a unit dose of fipronil ranging from 5 to 15 mg per kg of body weight.

**Patentansprüche**

1. Flüssige pharmazeutische Zusammensetzung, **dadurch gekennzeichnet, dass** diese umfasst:

    - 5-Amino-1-[2,6-dichlor-4-(trifluormethyl)phenyl]-4-(trifluormethylsulfinyl)-1H-pyrazol-3-carbonitril, das von 1 bis 20 g pro 100 ml Zusammensetzung darstellt, als Wirkstoff, und
    - Alpha-(tetrahydrofuranyl)-omega-hydroxypoly(oxy-1,2-ethandiyl) als Lösungsmittel.

2. Zusammensetzung nach Anspruch 1, **dadurch gekennzeichnet, dass** 5-Amino-1-[2,6-dichlor-4-(trifluormethyl)phenyl]-4-(trifluormethylsulfinyl)-1H-pyrazol-3-carbonitril von 5 bis 15 g pro 100 ml Zusammensetzung darstellt.

3. Zusammensetzung nach einem der vorherigen Ansprüche, **dadurch gekennzeichnet, dass** sie weiterhin einen oder mehrere Hilfsstoffe ausgewählt aus Tensiden, Verdickungsmitten, Farbstoffen, Duftstoffen und Antioxidationsmitteln umfasst.

4. Zusammensetzung nach Anspruch 3, **dadurch gekennzeichnet, dass** die Antioxidationsmittel ausgewählt sind aus Butylhydroxyanisol, Butylhydroxytoluol, Propylgallat, Ascorbylpalmitat, Rosmarinextrakten und Mischungen davon.

5. Zusammensetzung nach Anspruch 3 oder 4, **dadurch kennzeichnet, dass**, wenn vorhanden, das oder die Oxidationsmittel 0,005 bis 2 Gew.-% bezogen auf das Gesamtvolumen der Zusammensetzung darstellen.

6. Zusammensetzung nach einem der vorherigen Ansprüche, **dadurch gekennzeichnet, dass** diese einen oder mehrere zusätzliche Wirkstoffe gegen Parasiten umfasst.

7. Zusammensetzung nach Anspruch 6, **dadurch gekennzeichnet, dass** diese zusätzlichen Wirkstoffe gegen Parasiten ausgewählt sind aus Akariziden, Inhibitoren der Entwicklung von Flöhen und Zecken, Endoparasitiziden und Wirkstoffen gegen Phlebotome und Ektoparasiten bei Haustieren.

8. Verwendung einer flüssigen pharmazeutischen Zusammensetzung, wie sie in einem der vorherigen Ansprüche beschrieben ist, zur Herstellung eines antiparasitären veterinären Medikaments zur topischen Anwendung zur Verhinderung und/oder Behandlung von Flohbefall bei Haustieren.

9. Verwendung nach Anspruch 8, **dadurch gekennzeichnet, dass** das Medikament für Hunde oder Katzen bestimmt ist.

10. Verwendung nach Anspruch 8 oder 9, **dadurch gekennzeichnet, dass** das Medikament dazu bestimmt ist, der Katze in einer Menge von 0,3 bis 1,5 ml verabreicht zu werden.

11. Verwendung nach Anspruch 8 oder 9, **dadurch gekennzeichnet, dass** das Medikament dazu bestimmt ist, dem Hund in einer Menge von 0,3 bis 6,0 ml verabreicht zu werden.

12. Verwendung nach einem der Ansprüche 8 bis 11, **dadurch gekennzeichnet, dass** das Medikament dazu bestimmt ist, in einer Einheitsdosis Fipronil von 5 bis 15 mg pro kg Körpergewicht verabreicht zu werden.

EP 2 234 492 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0295117 A
- EP 0352944 A

- EP 0881881 A